(19) Europäisches Patentamt
European Patent Office
Office européen des brevets

(11) **EP 2 119 397 B1**

(12) **EUROPÄISCHE PATENTSCHRIFT**

(45) Veröffentlichungstag und Bekanntmachung des Hinweises auf die Patenterteilung:
**18.12.2013   Patentblatt 2013/51**

(51) Int Cl.:
***A61B 6/00*** *(2006.01)*

(21) Anmeldenummer: **08156293.6**

(22) Anmeldetag: **15.05.2008**

(54) **Bestimmung einer Kalibrier-Information für ein Röntgengerät**

Determining calibration information for an x-ray machine

Détermination d'une information de calibrage pour un appareil de radiologie

(84) Benannte Vertragsstaaten:
**DE FR GB**

(43) Veröffentlichungstag der Anmeldung:
**18.11.2009   Patentblatt 2009/47**

(73) Patentinhaber: **Brainlab AG**
**85622 Feldkirchen (DE)**

(72) Erfinder:
• **Essenreiter, Robert**
**81737, München (DE)**
• **Bertram, Michael**
**85570, Markt Schwaben (DE)**

• **Ringholz, Martin**
**85609, Aschheim (DE)**

(74) Vertreter: **Schwabe - Sandmair - Marx**
**Patentanwälte**
**Stuntzstraße 16**
**81677 München (DE)**

(56) Entgegenhaltungen:
EP-A- 1 313 065      DE-A1- 19 936 408
DE-A1- 19 963 440      DE-A1-102005 059 301
US-A- 5 901 199      US-B1- 6 484 049

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft die Bestimmung einer Kalibrier-Information für ein Röntgengerät, das insbesondere in der Medizin, insbesondere bei der bildgeführten Chirurgie (IGS für "image guided surgery") Anwendung finden kann.

Aus US 4,791,934 ist ein System für stereotaktische Chirurgie bekannt, bei der Computertomographie eingesetzt wird. Aus dreidimensionalen Daten werden zweidimensionale Bilder abgeleitet, um hierauf basierend die Lage eines chirurgischen Instruments zu führen.

Weiter wird auf DE 102 15 808 B4 verwiesen.

US-B1-6 484 049 offenbart ein Trackingsystem mit punktförmigen Markern, die in einem festgelegten Muster in einer Kalibrierungsanordnung für ein Fluoroskop abgebildet werden. In jeder fluoroskopischen Abbildung wird eine Untermenge der Marker identifiziert und auf diese Weise werden dann die geometrischen Parameter zur Kamerakalibration von den Lagen der abgebildeten Marker abgeleitet. Ferner wird offenbart, dass die Lagen der Marker eine einfache Berechnung der zur Charakterisierung der Kamera notwendigen Parameter ermöglicht. Dazu wird mit dem Fluroskop eine große Anzahl von Fluoro-Bildern angefertigt und ein CT-Bilddatensatz aus diesem rekonstruiert. Zu diesem Zweck wird ein Näherungsalgorithmus gemäß der Methode der kleinsten Quadrate verwendet, der einen Zusammenhang zwischen der Bewegungsebene der Röntgenquelle und den ermittelten Lage der Röntgenquelle zum Zeitpunkt der Bildgebung herstellt.

DE 10 2005 059301 A1 offenbart ein Verfahren zur Bestimmung der Akquisitionsgeometrie eines Bildgebungssystems aus einem Satz Kalibrierungsmatrizen. Ziel dieser Schrift ist es, eine mechanische Verformung des C-Bogens bei der Berechnung der Akquisitionsgeometrie des Systems zu berücksichtigen. Dabei wird ein Phantom verwendet, dessen Anordnung im Raum mit Genauigkeit bekannt sein muss, wobei ebenfalls eine gravitationsbedingte Verformung eines C-Arms ermittelt werden soll. Hierzu ist im C-Bogen ein dreiachsiger Beschleunigungsmesser befestigt, mit dem die Richtung des Schwerkraftfeldes relativ zum C-Bogen gemessen wird.

EP 1 313 065 A1 ist wie die letztgenannte Schrift auf eine schwerkraftempfindliche Röntgenaufnahmeeinrichtung gerichtet. Auch hier wird eine Vielzahl von Kalibrierungsaufnahmen angefertigt.

DE 199 36 408 A 1 offenbart ein fahrbares Röntgengerät und ein Verfahren zur Bestimmung von Projektionsgeometrien, bei dem aus einer Serie von 2D-Projektionen die Rekonstruktion wenigstens eines 3D-Bildes eines abgebildeten Objekts vorgenommen wird.

Aufgabe der Erfindung ist es, eine Kalibrier-Information für ein Röntgengerät, das einen 3D-Röntgenscan durchführt, zu bestimmen.

Vorstehende Aufgabe wird durch die Gegenstände der unabhängigen Ansprüche gelöst. Vorteilhafte Weiterbildungen gehen aus den abhängigen Ansprüchen hervor.

Pointer sind mit Markern versehene Zeigeeinrichtungen, die mittels der Kamera eines Navigationssystems detektiert werden können, um so die Lage des Pointers, insbesondere der Pointerspitze zu bestimmen. Auf diese Art und Weise kann die Lage von Punkten, insbesondere Landmarken in einer anatomischen Struktur in das Navigationssystem eingelesen werden.

Ein Vorteil der Erfindung liegt darin, dass das zeitaufwändige Verfahren des Einlesens von Daten mittels Pointer vermieden wird. Ein weiterer Vorteil der Erfindung liegt darin, dass ein Kalibrier-Objekt (zumindest dessen Ränder in einem (2D- und 3D-)Röntgenbild erkennbar sind) nicht mit Markern versehen werden muss, die sowohl optisch (durch eine Detektionseinrichtung eines Navigationssystems) als auch im Röntgenbild erkennbar sind. Dadurch kann das Kalibrier-Objekt kostengünstig gestaltet werden. Eine präzise Ausformung des Kalibrier-Objekts ist zumindest gemäß einer bevorzugten Variante der Erfindung nicht notwendig, sondern Form und/oder Abmessungen des Kalibrier-Objekts können beliebig und insbesondere gemäß der bevorzugten Variante der Erfindung unbekannt sein. Dennoch erlaubt das erfindungsgemäße Verfahren die Bestimmung der Kalibrier-Information, die Information über die Lagebeziehung und/oder räumliche Beziehung zwischen dem Röntgengerät-Bezugssystem und dem 3D-Scan-Bezugssystem umfasst und die insbesondere zum Navigieren benötigt wird und insbesondere mit Hilfe des Navigationssystems bestimmt wird. Die Kalibrier-Information umfasst vorzugsweise Information, die durch eine Transformation darstellbar ist. Diese Tranformation wird hierin Kalibrier-Transformation genannt. Die Kalibrier-Transformation umfasst insbesondere eine Transformation zwischen dem Röntgengerät-Bezugssystem und dem 3D-Scan-Bezugssystem. Die Kalibrier-Information kann durch mathematische Funktionen, Abbildungen oder Größen darstellbar sein, wie z.B. einen Satz von Vektoren, der die räumliche Beziehung zwischen dem Röntgengerät-Bezugssystem und dem 3D-Scan-Bezugssystem darstellt.

Bei der folgenden beispielhaften Beschreibung wird die Kalibrier-Transformation als Beispiel für eine Kalibrier-Information genannt.

Bei dem erfindungsgemäßen Verfahren wird ein 3D-Kalibrierdatensatz bereitgestellt. Der 3D-Kalibrierdatensatz wird durch einen dreidimensionalen Scan eines Kalibrier-Objekts mittels Röntgenstrahlen erstellt. Die so ermittelten Rohdaten werden vorzugsweise mit einer Datenverarbeitungseinrichtung verarbeitet, die aus den Rohdaten ein dreidimensionales Kalibrier-Modell errechnet, das das Kalibrier-Objekt dreidimensional darstellt. Dieses Kalibrier-Modell wird in einem Bezugssystem des 3D-Scans beschrieben. In diesem 3D-Scan-Bezugssystem ruht das Kalibrier-Modell, wobei sich beispielsweise eine Röntgeneinheit (z.B. ein C-Bogen) in dem 3D-Scan-Bezugssystem während des Scanvorgangs, insbesondere einer vorgegebenen Bewegungstrajektorie folgend, bewegt. Es gibt somit einen 3D-Scanpfad, entlang dem die Röntgeneinheit während des Scanvorgangs bewegt wird, um das Kalibrierobjekt aus verschiedenen Richtungen zu erfassen. Die so erstellten Aufnahmen aus verschiedenen Richtungen stellen Rohdaten dar, die dann, wie oben beschrieben, zu dem Kalibrier-Modell verarbeitet werden. Ein Abschnitt des 3D-Scanpfades, z.B. die Startposition der Röntgeneinheit, die diese zu Beginn des 3D-Scans einnimmt, ruht in einem Bezugssystem, das hierin Röntgengerät-Bezugssystem genannt wird.

Die Kalibrier-Transformation umfasst und insbesondere beschreibt eine Transformation zwischen dem Röntgengerät-Bezugssystem und dem 3D-Scan-Bezugssystem. "Umfassen" bedeutet hierin, dass sie auch durch ein Produkt bzw. eine Abfolge aus einer Transformation zwischen dem Röntgengerät-Bezugssystem und dem 3D-Scan-Bezugssystem und einer weiteren Transformation darstellbar ist. Die weitere Transformation ist z. B. eine Transformation zwischen einem anderen Bezugssystem (z.B. Navigationsbezugssystem, insbesondere Bezugssystem der Kamera des Navigationssystems oder Bezugssystem des Operationssaals, oder Bezugssystem einer detektierten Markereinrichtung), bei dem es sich nicht um das 3D-Scan-Bezugssystem handelt, und dem Röntgengerät-Bezugssystem und/oder eine Transformation zwischen dem 3D-Scan-Bezugssystem und dem anderen Bezugssystem.

Die hierin beschriebene Kalibrier-Transformation kann, wie später beschrieben wird, verwendet werden, um die relative Lage zwischen einem Gegenstand und einem anatomischen Modell zu bestimmen. Hierzu kann so vorgegangen werden, dass die Lage des Gegenstandes unter Verwendung der Kalibrier-Transformation in das 3D-Scan-Bezugssystem transformiert wird. Dies ist die bevorzugte Variante, die später näher ausgeführt wird. Es kann natürlich auch so vorgegangen werden, dass ein dreidimensionales anatomisches Modell unter Verwendung der Kalibriertransformation in ein Bezugssystem transformiert wird, in dem der Gegenstand ruht. Auch so ergibt sich die relative Lage zwischen dem Gegenstand und dem anatomischen Modell. Im Folgenden wird die Kalibrier-Transformation so beschrieben, dass sie eine Transformation von dem Röntgengerät-Bezugssystem in das 3D-Scan-Bezugssystem umfasst (erste Transformationsrichtung). Erfindungsgemäß soll aber auch der Fall umfasst sein, der die oben genannte zweite Variante betrifft, wonach die Kalibrier-Transformation eine Transformation von dem 3D-Scan-Bezugssystem zu dem Röntgengerät-Bezugssystem beschreibt (zweite Transformationsrichtung). Allgemein ausgedrückt, umfasst die erfindungsgemäße Kalibrier-Transformation somit eine Transformation zwischen dem Röntgengerät-Bezugssystem und dem 3D-Scan-Bezugssystem, wobei beide Transformationsrichtungen von dem Begriff Kalibrier-Transformation umfasst sein sollen. Bei der folgenden beispielhaften Beschreibung wird davon ausgegangen, dass der Begriff "Kalibrier-Transformation" rein beispielhaft die erste (und bevorzugte) Transformationsrichtung vom Röntgengerät-Bezugssystem zum 3D-Scan-Bezugssystem betrifft, so weit dies nicht anders explizit erläutert wird.

Weiter werden vorzugsweise Daten bereitgestellt, die die Aufnahmebedingungen von zweidimensionalen Röntgenbildern näher beschreiben. Hierzu werden Röntgenquellen-Relativlagedaten bereitgestellt, die Information über die Röntgenstrahlen-Abbildungsgeometrie enthalten, und die insbesondere es erlauben, die Lage einer Röntgenquelle relativ zu einer Röntgeneinheit-Markereinrichtung zu berechnen und/oder insbesondere es erlauben, den Verlauf der Röntgenstrahlen durch das Kalibrierobjekt hindurch relativ zur Lage der Röntgeneinheit-Markereinrichtung zu bestimmen. Die errechnete Lage der Röntgenquelle wird als der errechnete Schnittpunkt der Röntgenstrahlen definiert, die detektiert werden. Diese Lage kann, muss aber nicht mit der realen Lage der Röntgenstrahlquelle übereinstimmen. Die Lage der Röntgenquelle ergibt sich mathematisch, wenn man Röntgenbilder auswertet und Prinzipien der Lochkamera-Abbildung zugrunde legt.

Markereinrichtungen umfassen vorzugsweise passive oder aktive Marker, deren Lage zueinander bestimmt wird oder die in einer bekannten und vorgegebenen relativen Lage zueinander sind. Die passiven Marker reflektieren Strahlen oder Wellen, beispielsweise Infrarotstrahlen oder Ultraschallwellen, während die aktiven Marker diese aussenden. Die reflektierten oder ausgesandten Strahlen oder Wellen werden von der Detektionseinrichtung (des Navigationssystems), beispielsweise eine Kamera, die insbesondere zwei Detektionsmodule aufweist, detektiert. Aus den Detektionssignalen lässt sich die Lage der Markereinrichtung bestimmen. Dies wird insbesondere bei der zuvor erwähnten IGS eingesetzt.

Dem erfindungsgemäßen Verfahren wird weiter ein so genannter 2D-Kalibrierdatensatz bereitgestellt. Der 2D-Kalibrierdatensatz beschreibt mindestens ein zweidimensionales Röntgenbild, vorzugsweise zwei oder mehr Röntgenbilder. Die Daten eines einzigen zweidimensionalen Röntgenbildes genügen (gemäß einer ersten Variante) als Bestandteil des 2D-Kalibrierdatensatzes, falls das Kalibrier-Objekt bekannte Abmessungen und/oder Konturen und/oder Abstände zwischen Elementen hat, so dass sich aus den abgebildeten Abmessungen, Konturen oder Elementen und deren Abständen errechnen lässt, wo sich das Kalibrier-Objekt bei der Aufnahme befand. Da sich die Röntgenstrahlen ausgehend von der Röntgenquelle strahlkegelförmig ausbreiten, erscheint das abgebildete Kalibrier-Objekt umso größer auf dem Röntgenbild, je weiter das Kalibrier-Objekt von der Röntgenquelle entfernt ist. Da die Röntgenstrahlen-Abbildungsgeometrie von den Röntgenquellen-Relativtagedaten bekannt ist, kann aus der Größe des abgebildeten Kalibrier-Objekts (oder von Teilen des Kalibrier-Objekts) auf den Abstand des Kalibrier-Objekts zur Röntgenquelle geschlossen werden. Aus der Formänderung (z.B. quadratisches Teil wird rautenförmig abgebildet) kann eine Kippung (z.B. relativ zum Mittelstrahlengang von der Röntgenquelle zum Röntgendetektor) bestimmt werden. Der Mittelstrahlengang erzeugt den Bildpunkt in der Mitte des Röntgenbildes. Sein Verlauf ist vorzugsweise aus den Röiitgenquellen-Relativlagedaten bekannt. Diese zusätzlichen Informationen (Größe und/oder Formänderung) können genutzt werden, so dass sogar auf ein zweites Röntgenbild zur Bestimmung der Kalibrier-Transformation verzichtet werden kann. Im Folgenden wird, allerdings beispielhalt, die zweite Variante mit (mindestens) zwei zweidimensionalen Röntgenbildern als bevorzugte Variante beschrieben. Insbesondere wird bei dieser zweiten Variante eine höhere Genauigkeit erwartet. Beide Varianten können natürlich kombiniert werden, insbesondere um die Genauigkeit weiter zu erhöhen.

Die zweidimensionalen Röntgenbilder werden also vorzugsweise unter den vorgenannten (mindestens zwei) unterschiedlichen Aufnahmebedingungen erstellt, die vorzugsweise so gestaltet sind, dass die Rüntgenstralilkegel bei den jeweiligen Aufnahmen aus unterschiedlichen Richtungen das Registrierobjekt durchstrahlen. Vorzugsweise besteht ein Winkel von ungefähr 90° zwischen den Richtungen, beispielsweise liegt der bevorzugte Winkel zwischen 60 und 120°. Die Röntgenquelle nimmt also bei unterschiedlichen Aufnahmebedingungen unterschiedliche Relativlagen relativ zum Kalibrier-Objekt ein.

Wie zuvor erwähnt, werden Röntgenquellen-Relativlagedaten bereitgestellt, aus denen sich die relative Lage der Röntgeneinheit-Markereinrichtung zu der Röntgenquelle ergibt. Die relative Lage zwischen der Röntgenquelle und der Röntgeneinheit-Markereinrichtung ist zumindest näherungsweise konstant, kann sich aber für die verschiedenen Aufnahmebedingungen z.B. aufgrund einer Verformung des C-Bogens ändern. Dies kann dadurch berücksichtigt werden, dass die Röntgenquellen-Relativlagedaten für die verschiedenen Auihahmebedingungen verschiedene Daten umfassen, die Information enthalten, aus der sich die jeweilige, möglicherweise leicht unterschiedliche Röntgenstrahlen-Abbildungsgeometrie, insbesondere die relative Lage zwischen der Röntgeneinheit-Markereinrichtung und der Röntgenquelle bestimmen lässt. Vorzugsweise werden die Röntgenquellen-Relativlagedaten aus den zweidimensionalen Röntgenbildern bestimmt, indem diese ausgewertet werden. Vorzugsweise wird hierzu eine Röntgenquellen-Bestimmungshilfe eingesetzt, die sich bei der Aufnahme zumindest eines der zweidimensionalen Röntgenbilder (von dem Kalibrier-Objekt) und/oder bei einem separat aufgenommenem zweidimensionalen Röntgenbild im Strahlengang befindet und die eine bekannte relative Lage zur Röntgeneinheit-Markereinrichtung hat. Gemäß einer alternativen und einfacheren Ausführungsform ist die relative Lage zwischen der Röntgenquelle und der Röntgeneinheit-Markereinrichtung bekannt. Insbesondere kann davon ausgegangen werden, dass bestimmte vorbekannte Relativlagen für bestimmte Aufnahmebedingungen gegeben sind, so dass eine Röntgcnquellen-Bestimmungshilfe nicht erforderlich ist. Vorzugsweise wird jedoch die oben genannte Röntgenquellen-Bestimmungshilfe verwendet, insbesondere um eine höhere Genauigkeit zu erzielen. Insbesondere kann berücksichtigt werden, dass sich die Relativlagen im Laufe der Lebenszeit eines Röntgengeräts ändern können.

Die Röatgenquellen-Bestimmungshilfe umfasst vorzugsweise Strukturen oder Elemente, die in Richtung des Röntgcnstrahlenverlaufs voneinander in einem bekannten Maß beabstandet sind und im Röntgenbild nachweisbar sind. Aus den aus der Abbildung (Röntgenbild) bestimmbaren Abständen der Elemente und/oder Konturen der Röntgenquellen-Bestimmungshife lässt sich die Röntgenstrahlen-Abbildungsgeometrie bestimmen. Insbesondere lässt sich durch Rückprojektion von den abgebildeten Elementen bzw. Konturen über die bekannte Lage der Elemente und/oder Konturen der Röntgenquellen-Bestimmungshiife ein Projektionszentrum bestimmen, das der Lage der Röntgenquelle entspricht. Dadurch ist die Lage der Röntgenquelle bestimmt. Die Lage der Röntgenquelle wird insbesondere über die Lage des Projektionszentrums (Röntgenquelle) relativ zur Röntgeneinheit-Markereinrichtung beschrieben. Insbesondere werden die unterschiedlichen Lagen der Röntgenquelle, die sich für die unterschiedlichen Aufnahmebedingungen ergeben, im Röntgengerät-Bezugssystem (in dem z.B. die Startposition der Röntgeneinheit ruht) beschrieben.

Zur Bestimmung des Projektionszentrums und damit der Lage der Röntgenquelle aus den Röntgenbildern werden vorzugsweise mathematische Verfahren angewendet, die auf den Prinzipien einer Lochkamera (pin hole camera) beruhen. Hierzu wird auf folgende Veröffentlichungen verwiesen, die durch Bezugnahme in die Offenbarung mit aufgenommen sind:

1. "An Efficient and Accurate Camera Calibration Technique for 3D Machine Vision", Roger Y. Tsai, Proceedings of IEEE Conference on Computer Vision and Pattern Recognition, Miami Beach, FL, 1986, Seiten 364-374.

2. "A Versatile Camera Calibration Technique for High-Accuracy 3D Machine Vision Metrology Using Off-the-Shelf TV Cameras and Lenses", Roger Y. Tsai, IEEE Journal of Robotics and Automation, Vol. RA-3, No. 4, August 1987, Seiten 323-344. siehe auch unter http://www.cs.cmu.edu/~rgw/TsaiDesc.html

3. Veröffentlichung von Ziv Yaniv, "Fluoroscopic X-ray Image Processing and Registration for Computer-Aided Orthopedic Surgery".

Vorzugsweise werden beim Durchlaufen eines 3D-Scanpfades zur Bestimmung der Lage des Kalibrier-Modells und zur Bestimmung der Lage eines anatomischen Modells, wie dies später beschrieben wird, dieselben Startpositionen und/oder Endpositionen verwendet.

Die Röntgeneinheit-Markereinrichtung kann z.B. an einem Röntgenstrahldetektor oder an einer (realen) Röntgenstrahlquelle des Röntgengeräts angebracht sein. Der Röntgenstrahldetektor kann zusammen mit der Röntgenstrahlquelle bewegt werden, um die unterschiedlichen Aufnahmebedingungen (Aufnahmerichtungen) zu realisieren.

Ferner wird ein Röntgeneinheit-Datensatz bereitgestellt, der jeweils die Lage der Röntgeneinheit-Markereinrichtung für die verschiedenen Aufnahmebedingungen beschreibt. Beispielsweise kann diese Lage in einem bei der Berechnung der Kalibrier-Transformation verwendeten Bezugssystem, wie z.B. im Röntgengerät-Bezugssystem beschrieben werden. Alternativ und nicht im Rahmen der Erfindung kann diese Lage im später erwähnten Patienten-Bezugssystem oder auch in einem Navigations-Bezugssystem beschrieben werden. Durch den Röntgeneinheit-Datensatz wird dem erfindungsgemäßen Verfahren somit Information über die Lage der Röntgeneinheit-Markereinrichtung (insbesondere über deren relative Lage bei den verschiedenen Aufnahmebedingungen) bei der Erstellung der zweidimensionalen Röntgenbilder (des Kalibrier-Objekts) bereitgestellt. Diese Lage wird im Röntgengerät-Bezugssystem beschrieben.

Dem Verfahren steht nun Information über die relative Lage zwischen der Röntgeneinheit-Markereinrichtung und der Röntgenquelle für die verschiedenen Aufnahmebedingungen bei Erstellung der zweidimensionalen Röntgenbilder zur Verfügung. Außerdem ist die Lage der Röntgeneinheit-Markereinrichtung für die jeweiligen Aufnahmebedingungen bekannt. Somit lässt sich die jeweilige Lage der Röntgenquelle für die verschiedenen Aufnahmebedingungen relativ zueinander, vorzugsweise im Röntgengerät-Bezugssystem, aber beispielsweise auch im Navigations-Bezugssystem aus den Röntgenquellen-Relativlagedaten und dem Röntgeneinheit-Datensatz berechnen.

Dem Verfahren steht somit Information über die Aufnahmebedingungen, insbesondere die Lage der Röntgenquelle bei der Erzeugung der zweidimensionalen Röntgenbilder zur Verfügung. Mittels eines Anpassungsverfahrens, insbesondere eines mathematischen und/oder numerischen Anpassungsverfahrens, kann nun ermittelt werden, wie das Kalibrier-Objekt in einem Bezugssystem, vorzugsweise im Röntgengerät-Bezugssystem (aber z.B. auch im Navigations-Bezugssystem) liegen müsste, um die zweidimensionalen Röntgenbilder zu erzeugen. Hierzu kann insbesondere das Kalibrier-Modell herangezogen werden und z.B. im Röntgengerät-Bezugssystem (oder im Navigations-Bezugssystem) mittels einer Test-Transformation virtuell verlagert werden, insbesondere translatiert und/oder rotiert werden, bis sich bei virtueller Durchstrahlung des Kalibrier-Modells mit den Röntgenstrahlen von der Röntgenquelle die zweidimensionalen Röntgenbilder ergeben. Bei dieser Bestimmung der gültigen Test-Transformation nimmt die Röntgenquelle zum virtuellen Erzeugen der verschiedenen Röntgenbilder jeweils die unterschiedlichen Lagen ein. Die (virtuelle) Röntgenquelle hat hierbei jeweils eine der zuvor bestimmten Lagen z.B. im Röntgengerät-Bezugssystem oder im Navigations-Bezugssystem, die für die jeweilige Aufnahme der realen zweidimensionalen Röntgenbilder ermittelt wurde. Umfasst die Kalibrier-Transformation die Transformationsrichtung vom Röntgengerät-Bezugssystem zum 3D-Scan-Bezugssystem (erste Transformationsrichtung), so ist die Kalibrier-Transformation die Inverse der Test-Transformation. Ist die oben beschriebene alternative Variante der Kalibrier-Transformation gewählt, bei der die KalibrierTransformation die Richtung vom 3D-Scan-Bezugssystem zum Röntgengerät-Bezugssystem umfasst (zweite Transformationsrichtung), so entspricht die gültige Test-Transformation der Kalibrier-Transformation. Insgesamt lässt sich also mit der Kalibrier-Transformation die Lage des Kalibrier-Modells so transformieren, dass man eine optimale Anpassung erzielt. Insbesondere kann mittels der gültigen Test-Transformation die Lage des Kalibrier-Modells im 3D-Scan-Bezugssystem in diejenige Lage des Kalibrier-Modells im Röntgengerät-Bezugssystem transformiert werden, für die sich bei virtueller Durchstrahlung des Kalibrier-Modells (im Röntgengerät-Bezugssystem) virtuelle zweidimensionale Röntgenbilder ergeben, die optimal an die realen zweidimensionalen Röntgenbilder angepasst sind.

Ob eine Anpassung im gewünschten Umfang und somit eine Bestimmung der KalibrierTransformation mit der gewünschten Genauigkeit erzielt wurde, kann beispielsweise dadurch bestimmt werden, in welchem Umfang die virtuellen Röntgenbilder mit den realen Röntgenbildern übereinstimmen. Hierzu können beispielsweise die Lage von markanten Bereichen, beispielsweise Kanten oder Ecken des Kalibrier-Modells im virtuellen Röntgenbild mit der Lage der entsprechenden Kanten oder Ecken im realen Röntgenbild verglichen werden. Zum Beispiel mittels eines Least-Square-Fit-Verlahrens lässt sich eine Größe optimieren, die den Umfang der Übereinstimmung darstellt. Es können vorbestimmte Bereiche für diesen Umfang vorgegeben werden. Falls die Übereinstimmung innerhalb des vorgegebenen Bereichs liegt, kann die Übereinstimmung als gewünscht angesehen werden und somit die so bestimmte Kalibrier-Transformation als bestimmt angenommen werden.

Geht man davon ans, dass zur Bestimmung der Kalibrier-Transformation die Lage des Kalibrier-Modells mithilfe der Test-Transformationen variiert wird, indem beispielsweise ein Rotations- und/oder Translationsanteil variiert wird, so werden durch die Test-Transformationen Test-Lagen des Kalibrier-Modells erzeugt. Für die jeweilige Test-Lage wird dann vorzugsweise ein Satz von virtuellen Röntgenbildern erzeugt, die sich für die vorgegebenen Lagen der Röntgenquelle ergeben. Bei der vorgenannten Überprüfung der Übereinstimmung werden die so erzeugten virtuellen Röntgenbildern mit den realen Röntgenbildern verglichen, um so aus der Gesamtheit der Bilder einen Wert für die Übereinstimmung zu bestimmen. Ist die Übereinstimmung in einem vorbestimmten Umfang gegeben, so wird die Test-Transformation als gültig befunden und aus ihr die Kalibrier-Transformation bestimmt.

Wie erwähnt, umschreibt vorzugsweise der 2D-Kalibrierdatensatz nicht nur mindestens ein zweidimensionales Röntenbild des Kalibrier-Objekts sondern auch eine Abbildung der Röntgenquellen-Bestimmungshilfe, die von dem Röntgenstrahlkegel durchstrahlt wird und ein Muster in dem zweidimensionalen Röntgenbild ausbildet. Die Röntgenstrahlen durchstrahlen also bei der Erzeugung der zweidimensionalen Röntgenbilder nicht nur das Kalibrier-Objekt sondern vorzugsweise auch die Röntgenquellen-Bestimmungshilfe. Die Röntgenquellen-Bestimmungshilfe kann beispielsweise gitterförmig sein, wobei das Gitter ausgebildet ist, Röntgenstrahlen zu absorbieren. Vorzugweise sind mindestens zwei Gitter in einem vorbestimmten Abstand angeordnet, wobei die Gitter quer zur Ausbreitungsrichtung der Röntgenstrahlen sind. Auch können beispielsweise in einem für Röntgenstrahlen durchlässigen Material für Röntgenstrahlen undurchlässige Elemente wie z.B. Zylinder oder Kugeln (X-Ray-Marker) eingebettet sein, die wiederum Muster im zweidimensionalen Röntgenbild hinterlassen und vorzugsweise zumindest auch in Ausbreitungsrichtung der Röntgenstrahlen so beabstandet sind, dass sie im Röntgenbild nachgewiesen werden können. Das Muster kann regelmäßig oder unregelmäßig sein. Von dem Muster sind vorzugsweise geometrische Größen, insbesondere Abstände zwischen markanten Elementen des Musters (z.B. des Gitters) bekannt. Unter Verwendung der bereits oben erwähnten Prinzipien der Lochkamera wird basierend auf den bekannten Abständen zwischen markanten Musterelementen (z.B. Gitter·kreuzpunkte) und auf den zweidimensionalen Röntgenbildern vorzugsweise die Lage der Röntgenquellen, insbesondere des Projektionszentrums (der Röntgenquelle) berechnet. Auch können Verzerrungen und/oder Abbildungsfehler, die insbesondere durch die kissenförmige Oberfläche des Leuchtschirms im Bildwandler des Röntgenempfängers bedingt sind, in den zweidimensionalen Röntgenbildern korrigiert werden, um dann letztendlich ein möglichst abbildungsfehlerfreies und verzerrungsfreies Kalibrier-Modell mithilfe des (entzerrten) 2D-Kalibrierdatensatzes zu bestimmen. Insbesondere können die markanten Musterelemente beim Anpassungsverfahren zur Bestimmung der Kalibriertransformation verwendet werden, indem virtuell eine virtuelle Röntgenquellen-Bestimmungshilfe durchstrahlt wird, die sich in der selben Lage wie die reale Röntgenquellen-Bestimmungshilfe befindet und die selben Abmessungen hat. Insbesondere können also die virtuellen zweidimensionalen Röntgenbilder so berechnet werden, als ob die Röntgenquelle nicht nur das Kalibrier-Modell durchstrahlen würde sondern auch die in einer bekannten Lage befindliche Röntgenquellen-Bestimmungshilfe, die ein Bildmuster im Röntgenbild erzeugt. Wie bereits erwähnt, sind vorzugsweise geometrische Daten der Röntgenquellen-Bestimmungshilfe bekannt, die beispielsweise Abstände markanter Elemente der Röntgenquellen-Bestimmungshilfe oder die Größe und Abmessungen markanter Elemente, wie z.B. von Markerkugeln beschreiben. Mit Hilfe der geometrischen Daten der Röntgenquellen-Bestimmungshilfe kann ein entzerrtes Kalibrier-Modell berechnet werden. Außerdem kann hierauf basierend das Kalibrier-Modell noch skaliert werden, falls das Röntgengerät nicht bereits ausreichend genau skalierte Daten ausgibt.

Vorzugsweise wird gemäß einer Ausführungsform ein 3D-Messdatensatz bereitgestellt, der ein dreidimensionales anatomisches Modell einer anatomischen Struktur eines Patienten in einem Patienten-Bezugssystem beschreibt, die durch einen dreidimensionalen Röntgenscan (Mess-3D-Röntgenscan) erfasst wird. Die bestimmte Kalibrier-Transformation kann genutzt werden, um die Lage eines Gegenstandes relativ zur Lage des dreidimensionalen anatomischen Modells zu bestimmen. Eine gemäß dem obigen Verfahren bestimmte Kalibrier-Transformation kann demnach eingesetzt werden, um beispielsweise während einer Operation die Lage eines Gegenstands, z.B. Instrument oder Implantat relativ zu einer anatomischen Struktur zu bestimmen, ohne dass zusätzliche Schritte, wie z.B. die Erfassung der anatomischen Struktur mit einem Pointer oder die Aufnahme zweidimensionaler Röntgenbilder erforderlich sind.

Bei dem oben beschriebenen Verfahren zur Bestimmung der Lage des Gegenstandes relativ zur Lage des dreidimensionalen anatomischen Modells wird vorzugsweise das Röntgengerät-Bezugssystem genutzt, da man annehmen kann, dass sich die Lage des 3D-Scan-Bezugssystems relativ zu dem Röntgengerät-Bezugssystem seit der Ermittlung der Kalibriertransformation nicht geändert hat. Nutzt man beispielsweise das Navigations-Bezugssystem, so kann sich eine Lageänderung des Röntgengeräts seit der Bestimmung der Kalibrier-Transformation ergeben haben. Eine Änderung der Lage des Röntgengeräts würde eine Änderung der Lage des 3D-Scanpfades bedeuten. Der 3D-Scanpfad während der Erfassung des Kalibrier-Objekts hätte also eine andere Lage als der 3D-Scanpfad während der Erfassung der anatomischen Struktur. Mit anderen Worten das Röntgengerät-Bezugssystem ändert also seine Lage im Navigations-Bezugssystem. Um dies zu berücksichtigen, werden insbesondere in diesem Fall, vorzugsweise Scanpfad-Relativlagedaten bereitgestellt, die eine Lageänderung des 3D-Scanpfades im Navigations-Bezugssystem beschreiben, die zwischen dem Kalibrier-3D-Röntgenscan und dem Mess-3D-Röntgenscan stattgefunden hat. Eine derartige Lageänderung kann beispielsweise erfasst werden, indem die Lage der Röntgeneinheit-Markereinrichtung bei gleicher Stellung der Röntgeneinheit (z.B. Startposition des Scanvorganges) innerhalb des 3D-Scanpfades verglichen wird. Auch kann zusätzlich eine Markereinrichtung am Röntgengerät angebracht werden, die vorzugsweise während des Röntgenscans die Lage nicht ändert. Durch die Bestimmung der Lage dieser Markereinrichtung während des Kalibrier-3D-Röntgenscans und während des Mess-3D-Röntgenscans kann die Lageänderung des 3D-Scanpfades erfasst werden. Diese Lageänderung wird vorzugsweise durch eine Transformation ("Navi-Scan"-Transformation) beschrieben, die beispielsweise Rotations- und/oder Translationselemente umfasst. Diese "Navi-Scan"-Transformation beschreibt also die Lageänderung des Röntgengerät-Bezugssystems im Navigations-Bezugssystem. Kombiniert man diese Transformation mit der Transformation zwischen dem Röntgengerät-Bezugssystem und dem 3D-Scan-Bezugssystem ("Röntgen-Scan"-Transformation), so ergibt sich auch ein Beispiel für die Kalibrier-Transformation. Dieses Beispiel ist eine Transformation zwischen dem Navigations-Bezugssystem und dem 3D-Scan-Bezugssystem und umfasst, wie vorstehend ausgeführt, die Transformation vom Röntgengerät-Bezugssystem zum 3D-Scan-Bezugssystem ("Röntgen-Scan"-Transformation). Sowohl die Navi-Scan-Transformation als auch die Röntgen-Scan-Transformation sind Beispiele für eine Kalibrier-Transformation.

Ein mögliches Navigations-Bezugssystem ist beispielsweise ein Bezugssystem, in dem der Operationssaal ruht oder in dem eine Detektionseinrichtung (z.B. Kamera) des Navigationssystems ruht. Die Lage, die ein Gegenstand, z.B. ein Körperteil oder ein Instrument im Navigations-Bezugssystem einnimmt, kann mittels der Navi-Scan-Transformation in die Lage umgerechnet werden, die der Gegenstand im 3D-Scan-Bezugssystem einnimmt. Die Lage, die ein Gegenstand im Röntgengerät-Bezugssystem einnimmt, kann mittels der Röntgen-Scan-Transformation in die Lage umgerechnet werden, die der Gegenstand im 3D-Scan-Bezugssystem einnimmt.

Der 3D-Messdatensatz beschreibt die Lage des dreidimensionalen anatomischen Modells im 3D-Scan-Bezugssystem. Wird, wie oben beschrieben, die Lage des Gegenstands ebenfalls im 3D-Scan-Bezugssystem mithilfe der Kalibrier-Transformation berechnet, so erhält man die Lage des Gegenstands relativ zu dem dreidimensionalen anatomischen Modell, also beispielsweise die Lage eines Instruments relativ zu dem dreidimensionalen anatomischen Modell, beispielsweise einer Körperstruktur, wie dem Femur, wobei dies zur IGS genutzt werden kann.

In der Praxis wird es häufig auftreten, dass die anatomische Struktur nach der Erzeugung des 3D-Messdatensatzes ihre Lage ändert, insbesondere ihre Lage im Röntgengerät-Bezugssystem und/oder Navigations-Bezugssystem ändert. Um dennoch die aktuelle Lage eines Gegenstandes, z.B. Instruments relativ zu dem dreidimensionalen anatomischen Modell der aktuellen Situation entsprechend berechnen zu können, werden vorzugsweise Gegenstandsdaten bereitgestellt. Die Gegenstandsdaten umfassen vorzugsweise Gegenstands-Röntgeneinheit-Daten und/oder Gegenstands-Patienten-Daten. Die Gegenstands-Röntgeneinheit-Daten beschreiben die Lage des Gegenstands im Röntgengerät-Bezugssystem, also beispielsweise relativ zu der Röntgeneinheit-Markereinrichtung. Die Gegenstands-Patienten-Daten beschreiben die Lage eines Gegenstands in einem Patienten-Bezugssystem, also z.B. relativ zu einer ortsfest mit der anatomischen Struktur verbundenen Patienten-Markereinrichtung. Die Verwendung der Gegenstands-Patienten-Daten hat gegenüber der Verwendung der Gegenstands-Röntgeneinheit-Daten den Vorteil, dass bei der Bestimmung der Lage des Gegenstands relativ zum dreidimensionalen anatomischen Modell das Röntgengerät auch vollständig entfernt sein kann. Die Röntgeneinheit-Markereinrichtung muss also nicht mehr detektierbar sein. Ist sie noch detektierbar, so kann zu jedem Zeitpunkt die relative Lage zwischen dem Gegenstand und der Röntgeneinheit-Markereinrichtung bestimmt werden, um so die Gegenstands-Röntgeneinheit-Daten zu aktualisieren. Ist also die Lage des Gegenstands im Röntgengerät-Bezugssystem aus dem Gegenstands-Röntgeneinheit-Daten bekannt, so kann durch Anwendung der Kalihrier-Transformation die Lage des Gegenstands relativ zum dreidimensionalen anatomischen Modell bestimmt werden. Im Folgenden wird die Ausführungsform näher erläutert, bei der basierend auf den Gegenstands-Patienten-Daten die Lage des Gegenstands relativ zu dem dreidimensionalen anatomischen Modell bestimmt wird.

Bei der Patienten-Markereimichtung handelt es sich beispielsweise um einen Referenzstern, der an der anatomischen Struktur des Patienten, wie z.B. an einem Knochen des Patienten befestigt ist. Da der Gegenstand vorzugsweise ebenfalls mit einer Markereinrichtung versehen ist, kann somit das Navigationssystem die relative Lage zwischen dem Gegenstand und der Patienten-Markereinrichtung und somit die erwähnten Gegenstands-Patienten-Daten bestimmen. Weiter werden vorzugsweise Markerrelativlagedaten bereitgestellt, die die relative Lage der Patienten-Markereinrichtung zu der Röntgeneinheit-Markereinrichtung oder zu einer anderen Markereinrichtung, die in dem Röntgengerät-Bezugssystem ruht, beschreiben. Die relative Lage kann in dem Röntgengerät-Bezugssystem (oder in einem anderen Bezugssystem, beispielsweise dem Navigations-Bezugssystem) beschrieben werden. Basierend auf den Markerrelativlagedaten wird vorzugsweise eine Mess-Transformation berechnet. Die Mess-Transformation beschreibt insbesondere eine Transformation von dem Patienten-Bezugssystem, in dem die Patienten-Markereinrichtung ruht, zu dem Röntgengerät-Bezugssystem. Wendet man also die Mess-Transformation auf die Lage eines Gegenstands an, die insbesondere in dem Patienten-Bezugssystem beschrieben ist, so erhält man die Lage des Gegenstands insbesondere im Röntgengerät-Bezugssystem. Wendet man hierauf nun die Kalibrier-Transformation (genauer die Röntgen-Scan-Transformation) an, so ergibt sich die Lage des Gegenstands im 3D-Scan-Bezugssystem. Diese Lage ist unabhängig von einer relativen Bewegung der anatomischen Struktur relativ zum Röntgengerät, da die Gegenstands-Patienten-Daten im Bezugssystem der Patienten-Markereinrichtung beschrieben sind. Vorzugsweise wird also die Mess-Transformation mit der Kalibrier-Transformation kombiniert, und die kombinierte Transformation (auch Registrier-Transformation genannt) erlaubt die Bestimmung der Lage des Gegenstands relativ zu dem dreidimensionalen anatomischen Modell auch bei relativen Bewegungen der anatomischen Struktur (und somit des zugeordneten anatomischen Modells) relativ zum Röntgengerät nach Erstellung des 3D-Messdatensatzes.

Vorzugsweise beschreibt der 3D-Kalibrierdatensatz ein Kalibrier-Objekt, das insbesondere eine unregelmäßige Form aufweist, die in zweidimensionalen Röntgenbildern Konturen zeigt. Vorteilhaft hieran ist, dass im Gegensatz zu einer regelmäßigen Kontur, wie z.B. einem Würfel oder Zylinder keine Symmetrieeigenschaften des Registrierobjekts gegeben sind, die eine klare Bestimmung der Aufnahtnerichtung behindern oder unmöglich machen. Deshalb werden unsymmetrische Registrierobjekte, insbesondere ohne Symmetrieebene, Symmetrieachse oder Symmetriepunkt bevorzugt.

Die vorliegende Erfindung bezieht sich weiterhin auf ein Programm, das, wenn es auf einem Computer ausgeführt wird oder darin geladen wird, den Computer veranlasst, das oben beschriebene Verfahren durchzuführen, insbesondere die Daten in der oben beschriebenen Weise zu verarbeiten.

Das vorgenannte Programm kann insbesondere auf einem Datenspeichermedium gespeichert sein, wie z.B. CD, DVD, ROM-Speicherkarten, etc. Das Programm kann auch über eine digitale Signalwelle, beispielsweise per Download übertragen werden. Das vorgenannte Programm wird also insbesondere durch ein Datenspeichermedium oder eine Signalwelle verkörpert.

Die Erfindung ist weiter auf eine Datenverarbeitungsvorrichtung, insbesondere einen Computer gerichtet, der das vorgenannte Programm umfasst, auf dem also das Programm geladen ist oder läuft.

Weiter ist die Erfindung auf ein Navigationssystem gerichtet, wie es beispielsweise bei der bildgeführten Navigation bei Operationen eingesetzt wird. Das Navigationssystem umfasst vorzugsweise die vorgenannten Datenverarbeitungsvorrichtung und zusätzlich eine Detektionseinrichtung, die dazu in der Lage ist, Markereinrichtungen zu detektieren. Insbesondere ist sie zur Detektion der Markereinrichtungen geeignet, die eingesetzt werden, um die Daten zu erzeugen, die dem vorgenannten Verfahren bereitgestellt werden bzw. die durch das vorgenannte Programm verarbeitet werden. Insbesondere wird durch die Detektionseinrichtung die Röntgeneinheit-Markereinrichtung detektiert und werden die Detektionssignale der Datenverarbeitungsvorrichtung zugeführt. Vorzugsweise detektiert die Detektionseinrichtung weiter die Patienten-Markereinrichtung und/oder eine weitere am Röntgengerät angebrachte Markereinrichtung, sowie insbesondere eine an einem Gegenstand (z.B. Instrument) angebrachte Markereinrichtung.

Das Navigationssystem wird vorzugsweise mit einem Röntgengerät kombiniert oder umfasst dieses, das die Röntgeneinheit umfasst, die zur Erzeugung des 3D-Kalibrierdatensatzes, des 2D-Kalibrierdatensatzes und insbesondere des 3D-Messdatensatzes eingesetzt wird. Die Röntgeneinheit ist zur Übermittlung der erfassten Röntgensignale vorzugsweise mit der Datenverarbeitungseinrichtung verbunden, um so die vorgenannten Datensätze zu erzeugen, um diese letztendlich dem Programm oder Verfahren zur Verfügung stellen zu können.

Bei der folgenden detaillierten Beschreibung werden weitere Vorteile und Merkmale der Erfindung offenbart, wobei verschiedene Merkmale unterschiedlicher Ausführungsformen miteinandcr kombiniert werden können.

Figur 1 zeigt eine Ausführungsform des erfindungsgemäßen Navigationssystems mit der Röntgeneinheit in der horizontalen Lage.
Figur 2 zeigt die Ausführungsform der Figur 1 mit der Röntgeneinheit in der vertikalen Lage.
Figur 3 zeigt die Ausführungsform der Figur 1 mit Patienten.

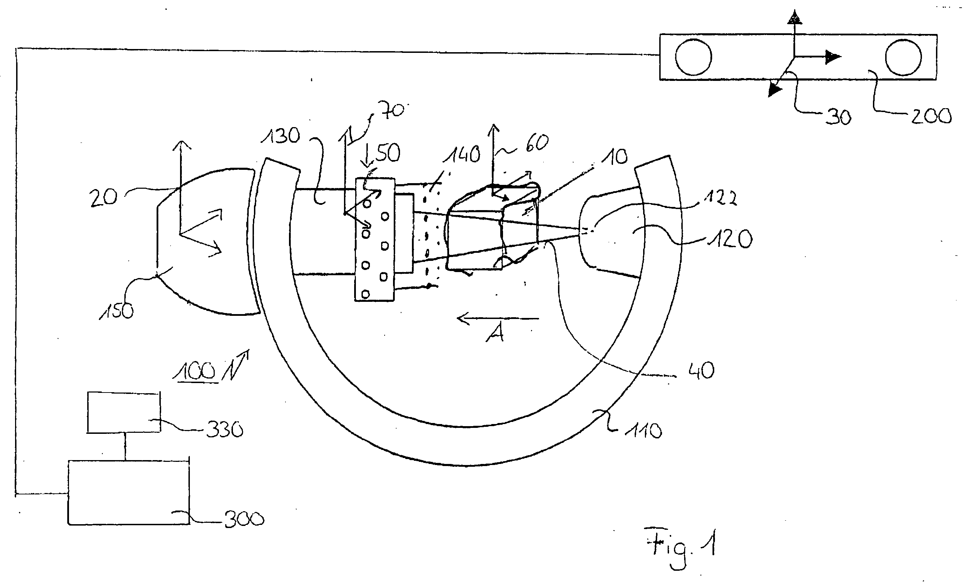

Figur 1 zeigt einen erfindungsgemäßen Aufbau. Ein Röntgengerät 100, genauer ein C-Bogen-Röntgengerät 100 besteht aus einem ruhenden Teil 150, der sich bei einem dreidimensionalen Scan nicht relativ zu einer Kamera 200 eines Navigationssystems bewegt. Weiter umfasst das Röntgengerät 100 eine bewegliche Röntgeneinheit 110 mit einer Röntgenstrahlquelle 120 und einen Röntgenlichtdetektor 130. An dem Röntgenlichtdetektor 130 ist ein so genanntes Registrierungskit 50 angebracht, das als Röntgeneinheit-Markereinrichtung dient. Ein kegelförmiger Röntgenstrahl 40 geht von einem Punkt 122 aus, der eine Röntgenquelle 122 darstellt, die dadurch definiert ist, dass sich in dem Punkt 122 Geraden, die die Röntgenstrahlen 40 darstellen, schneiden. Die Röntgenstrahlen werden von dem Röntgenlichtdetektor 130 erfasst. Ein Röntgengitter 140, das als Röntgenquellen-Bestimmungshilfe dient, wird von dem Röntgenstrahlkegel 40 durchstrahlt, so dass das Röntgengitter 140 vom Röntgenlichtdetektor 130 bildlich erfasst wird. Das Muster des Röntgengitters 140 ist also in der von dem Röntgenlichtdetektor erfassten Aufnahme erkennbar. Das Röntgengitter 140 umfasst vorzugsweise zwei Gitter, die in Ausbreitungsrichtung der Röntgenstrahlen (z.B. in Richtung der Mittelachse des Röntgenstrahlkegels) voneinander im bekannten Umfang beabstandet sind. Die räumliche Beziehung zwischen dem Röntgengitter 140 und dem Kit 50 ist bekannt. Insbesondere ist auch die relative räumliche Beziehung zwischen dem Kit 50 und den Gittern des Röntgengitters 140 bekannt. Aus den bekannten Abstandsbeziehungen zwischen den zwei (oder mehr) Gittern des Röntgengitters 140 und/oder zwischen Markerelementen des Röntgengitters, die insbesondere in Ausbreitungsrichtung der Röntgenstrahlen voneinander beabstandet sind, und durch Auswertung der Abstandsbeziehungen zwischen den Gitterelementen oder Markerelementen in dem zweidimensionalen Röntgenbild lässt sich bestimmen, wie die Röntgenstrahlen bei der Aufnahme sich ausgebreitet haben. Durch Projektion, insbesondere durch Anwendung der Lochkamera-Prinzipien lässt sich somit die Lage der Röntgenquelle 122 insbesondere relativ zum Kit 50 bestimmen.

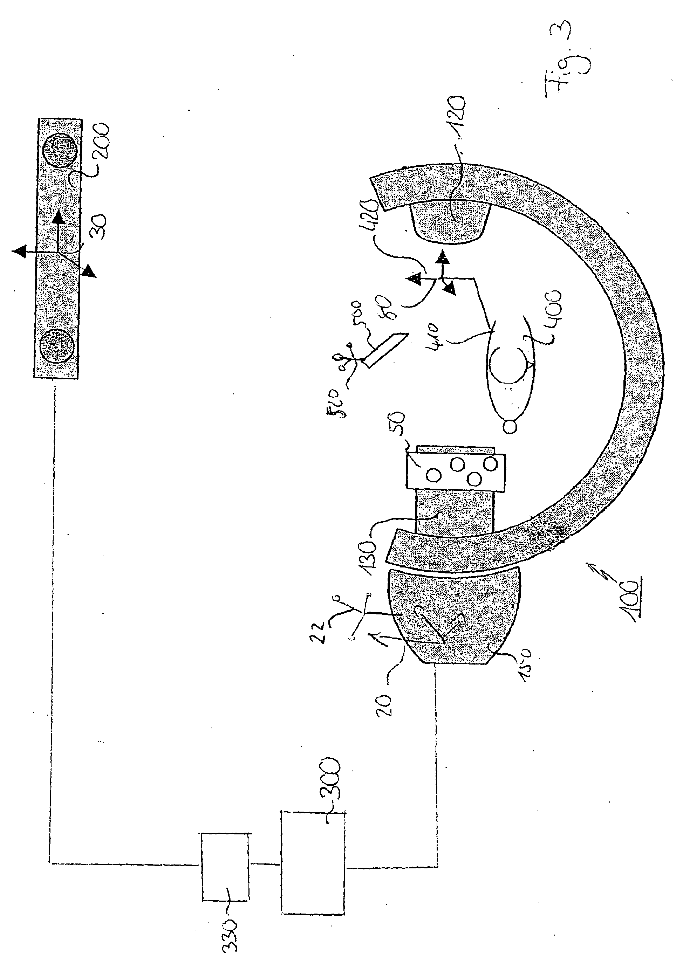

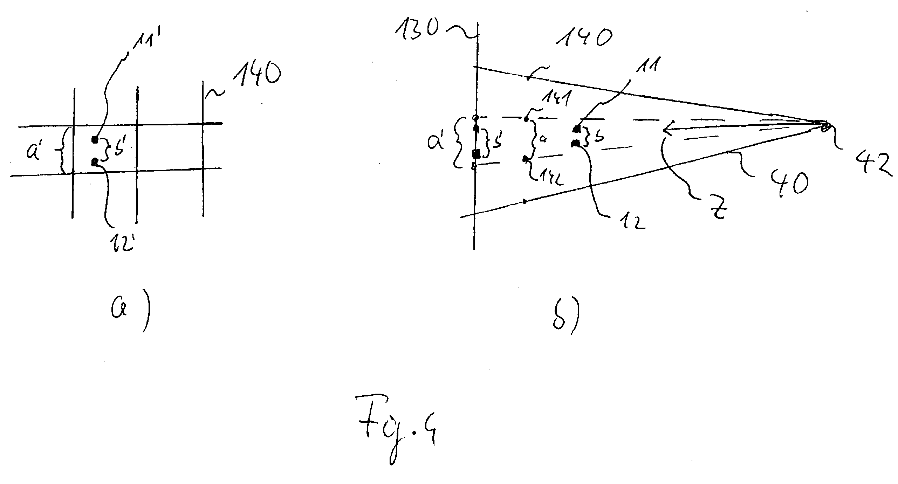

An einem Instrument 500 (siehe Figur 3) ist vorzugsweise ebenfalls eine Markereinrichtung angebracht, die durch die Detektionseinrichtung des Navigationssystems detektiert wird. Beschreibt man die Transformationen, die erforderlich sind, um die Position des Instruments 500 im 3D-Scan-Bezugssystem darzustellen, so ergibt sich folgende Situation:

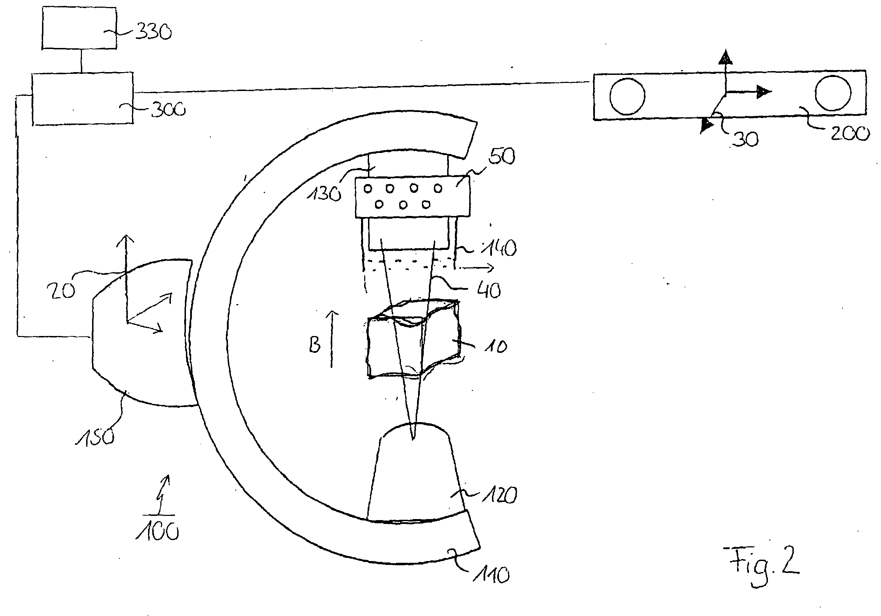

$M_{70-60}$: Diese Matrix beschreibt eine Kalibrier-Transformation von dem Röntgengerät-Bezugssystem 70, in dem das Kit 50 z.B. in seiner Startposition ruht, zu dem 3D-Scan-Bezugssystem 60. Diese Kalibrier-Transformation wurde gemäß dem oben beschriebenen Verfahren bestimmt. So wurden von dem Kalibrier-Objekt 10 zwei zweidimensionale Bilder in den in Figur 1 und Figur 2 gezeigten Stellungen erstellt, und es wurde ein 3D-Scan des Kalibrier-Objekts 10 durchgeführt. Bei den zweidimensionalen Röntgenbildern, die wie in Figuren 1 und 2 gezeigt jeweils erstellt wurden, erfolgt vorzugsweise die Aufnahme mit dem Röntgengitter 140. Die so erhaltenen zweidimensionalen und dreidimensionalen Daten werden in der bereits oben beschriebenen Art und Weise verarbeitet, um die Lagebeziehung zwischen dem 3D-Scan-Bezugssystem 60 und dem Röntgengerät-Bezugssystem 70 bestimmen zu können. Somit wird letztendlich die Kalibrier-Transförmation, genauer die Röntgen-Scan-Transformation bestimmt.

$M_{80-60}$: Diese Matrix beschreibt eine so genannte Registrierungs-Transformation vom Patienten-Bezugssystem 80, in dem der Referenzstern 420 ruht (siehe Figur 3), zu dem 3D-Scan-Bezugssystem 60.

$M_{80-70}$: Diese Matrix beschreibt eine Mess-Transformation von dem Patienten-Bezugssystem 80, in dem der Referenzstern 420 ruht, zu dem Röntgengerät-Bezugssystem 70.

Die Registrierungs-Matrix $M_{80-60}$ berechnet sich wie folgt:

$$M_{80-60} = M_{70-60} * M_{80-70}$$

Es ergibt sich also durch Multiplikation der Matrizen $M_{70-60}$ mit $M_{80-70}$.

Beobachtet man nun ein Instrument 500 mit einem Referenzstern 520 durch die Kamera 200 des Navigationssystems, so lässt sich die Lage des Instruments 500 im Bezugssystem 80 des Referenzsterns 420 (also die Gegenstands-Patienten-Daten) berechnen. Die Berechnung erfolgt basierend auf der detektierten Lage des Referenzsterns 520 und aufgrund der bekannten relativen Lage zwischen dem Referenzstern 520 und dem Instrument 500 oder eines Teil des Instruments, wie z.B. der Instrumentenspitze, und basierend auf der detektierten Lage des Referenzsterns 420.

Vorzugsweise wird basierend auf dem erfindungsgemäßen Verfahren die Lage des Instruments 500 relativ zu dem dreidimensionalen anatomischen Modell der anatomischen Struktur 410 berechnet. Hierzu kann beispielsweise ein Vektor $V_{80}$, der beispielsweise die berechnete Lage der Spitze des Instruments 500 im Patienten-Bezugssystem 80 (des Referenzsterns 420) darstellt, mit der oben genannten Matrix $M_{80-60}$ wie folgt multipliziert werden:

$$V_{60} = M_{80-60} * V_{80}$$

Der sich ergebende Vektor $V_{60}$ beschreibt dann die Position des Spitze des Instruments im 3D-Scan-Bezugssystem 60. Außerdem ist die Lage des dreidimensionalen Modells der anatomischen Struktur im 3D-Scan-Bezugssystem aus dem 3D-Messdatensatz bekannt. Somit kann bestimmt werden, wie die Spitze des Instruments 500 relativ zu dem dreidimensionalen Modell der anatomischen Struktur liegt. Diese Relativlage kann insbesondere in Echtzeit auf einem Bildschirm 330 des Navigationssystems angezeigt werden. Diese Relativlage wird auch durch eine Bewegung des Röntgengeräts relativ zur anatomischen Struktur nicht beeinflusst, so dass das Instrument 500 relativ zum anatomischen Modell auch ohne weitere Röntgenaufnahmen navigiert werden kann.

Um die Lage der Röntgenquelle, genauer des Projektionszentrums der Strahlen relativ zu dem Kit 50 zu bestimmen, umfasst das Röntgengitter 140 vorzugsweise mehrere Elemente (X-Ray-Marker), z.B. Kugeln auf vorzugsweise mindestens zwei Ebenen. Vorzugsweise sind mindestens sechs Kugeln auf mindestens zwei Ebenen bekannt. Das heißt die Lage dieser Kugeln relativ zum Kit 50 und somit im Bezugssystem 70 ist vorzugsweise bekannt. Durch die aus dem Stand der Technik bekannte Anwendung des Lochkameraprinzips (pin hole) lässt sich aus dieser bekannten Lage der Markerkugeln und/oder aus den Abständen der Markerkugeln sowie aus der relativen Lage und/oder den Abständen der abgebildeten Markerkugeln, die den zweidimensionalen Röntgenbildern zu entnehmen sind, die Lage eines Projektionszentrums bestimmen. Hierzu wird auf die bereits oben erwähnte Literatur verwiesen.

Die Richtung, in der die Aufnahme erfolgt, ist in Figur 1 mit A und einem Pfeil gekennzeichnet. Die Richtung weist von der Röntgenquelle zum Röntgenlichtdetektor und liegt im gezeigten Beispiel in der Horizontalen. Das Kalibrier-Objekt 10 ist in Figur 1 so gezeigt, dass es eine in etwa würfelförmige Gestalt hat. Vorzugsweise ist es jedoch unregelmäßig ausgeformt. Beispielsweise kann man als Kalibrier-Objekt ein Modell einer menschlichen Wirbelsäule nehmen. Vorzugsweise hat das Kalibrier-Objekt aber nicht die Gestalt eines geometrischen Grundkörpers, wie beispielsweise Würfel, Quader, Zylinder, Kugel oder Pyramide sondern ist asymmetrisch geformt (ohne Symmetrieebene, -achsen oder -punkte).

Als erstes wird beispielsweise das Kalibrier-Objekt 10 durch einen dreidimensionalen Scan erfasst, wie dies durch die Figuren 1 und 2 angedeutet ist. Die Figur 1 zeigt dabei beispielsweise den Beginn des dreidimensionalen Scanvorgangs, wobei der Röntgenstrahlkegel horizontal verläuft (0°-Stellung). Von dort ausgehend rotiert der Röntgenstrahlkegel in etwa um eine Achse, die durch den gedachten Mittelpunkt des kreisförmigen C-Bogens verläuft. Die Rotation verläuft beispielsweise über die in Figur 2 gezeigte Stellung, in der die Röntgenstrahlkegel 40 vertikal verläuft (90°-Stellung) bis zu einer nichtgezeigten Stellung, die z.B. eine 150° - 180° Drehung gegenüber der in Figur 1 gezeigten Stellung darstellt. Die Richtung der Röntgenaufnahme ist mit B und einem Pfeil gekennzeichnet. Die erfassten Bilddaten werden einer Datenverarbeitungseinrichtung zugeführt, die hieraus den 3D-Kalibrierdatensatz erstellt, der dreidimensional das Kalibrier-Objekt darstellt. Das Kalibrier-Modell wird in dem 3D-Scan-Bezugssystem 60 dargestellt.

Der dreidimensionale Scan wird vorzugsweise ohne das Röntgengitter 140 durchgeführt. Basierend auf den Scandaten wird der 3D-Kallbrierdatensatz und somit das Kalibrier-Modell berechnet. Zusätzlich zu dem dreidimensionalen Scan (d.h. vor oder nach dem dreidimensionalen Scan) werden vorzugsweise zwei zweidimensionale Röntgenbilder von dem Kalibrier-Objekt erfasst. Dabei ändert das Kalibrier-Objekt 10 vorzugsweise seine Lage nicht. Es hat also dieselbe Lage, wie während des dreidimensionalen Röntgenscans. Bei den zwei zweidimensionalen Röntgenbildern handelt es sich vorzugsweise um Fluoroskopiebilder. Die zweidimensionalen Röntgenbilder werden vorzugsweise mit montiertem Röntgengitter 140 getätigt. Sie bilden die Basis für den 2D-Kalibrierdatensatz. Weiter wird vorzugsweise ein so genannter Bildverstärker ("image intensifier) verwendet, der Teil des C-Arms ist, um die Empfindlichkeit des Röntgenlichtdetektors zu steigern. Die zwei zweidimensionalen Röntgenbilder werden vorzugsweise aus verschiedenen Richtungen relativ zum Registrierobjekt gemacht.

Vorzugsweise sind die Richtungen schräg und insbesondere senkrecht, wie in dem in Figur 1 und 2 gezeigten Beispiel. Die zwei zweidimensionalen Röntgenbilder können also beispielsweise aus der Richtung A und der Richtung B getätigt werden.

Nachdem die zwei zweidimensionalen Bilder und der dreidimensionale Scan von dem Kalibrier-Objekt erfasst worden sind, wird ein Anpassungsverfahren (Matchingverfahren) durchgeführt, um zu bestimmen, wie das Kalibrier-Modell, das das Kalibrier-Objekt zweidimensional darstellt, zwischen Röntgenstrahlquelle 120 und Röntgenstrahldetektor 130 liegen müsste, um die zwei zweidimensionalen Bilder zu erzeugen. Wie ein derartiges Anpassungsverfahren durchgeführt wird, wurde bereits oben erläutert. Es wird auch auf die US 4,791,934 verwiesen.

Beim Anpassungsverfahren können also virtuelle zweidimensionale Abbildungen aus dem Kalibrier-Modell erzeugt werden. Die Lage des Kalibrier-Modells relativ zur Röntgenquelle wird variiert, beispielsweise mittels einer Translations- und/oder Rotationstransformation, die als Test-Transformation dient. Vorzugsweise werden zwei virtuelle zweidimensionale Röntgenbilder erstellt, die mit zwei Röntgenquellen erzeugt werden, die die relative räumliche Lage haben, wie sie bei der Aufnahme der zwei reellen zweidimensionalen Röntgenbilder vorgelegen haben. Die relative Lagebeziehung der Richtungen A und B und/oder der Röntgenquelle kann insbesondere aus der relativen Lagebeziehung des Kits 50 bei den beiden Aufnahmen (Figur 1 und Figur 2) bestimmt werden, die durch die Kamera 200 erfasst wird. Dadurch dass die relative Lagebeziehung für den Anpassungsvorgang als vorgegeben angenommen wird, kann der Anpassungsvorgang zur Bestimmung der Registrier-Transformation schneller durchgeführt werden.

Wie bereits ausgeführt, erlaubt insbesondere der Einsatz des Röntgengitters die Bestimmung der Röntgenquelle, also insbesondere der Lage der Röntgenquelle (Projektionszentrum der Röntgenstrahlen). Außerdem kann es bei einer Skalierung des Kalibrier-Modells genutzt werden. Die erfindungsgemäße Kalibrier-Transformation kann somit nicht nur eine Lagebeziehung zwischen dem Kalibrier-Modell im 3D-Scan-Bezugssystem 60 und dem Kalibrier-Modell im Röntgengerät-Bezugssystem 70 oder dem Navigations-Bezugssystem 30 beschreiben sondern zusätzlich erlaubt sie insbesondere eine Überprüfung der Skalierung, wie sie von dem Röntgengerät ausgegeben wird, und/oder eine neue Skalierung, die sich z.B. durch eine Vergrößerung oder Verkleinerung und/oder Entzerrung des Kalibrier-Modells ausdrücken lässt. Das Kit 50 ruht in dem Röntgengerät-Bezugssystem 70 in der in Figur 1 gezeigten Startposition des Scanvorganges. Das Kalibrier-Modell ruht in dem 3D-Scan-Bezugssystem 60.

Der zuvor beschriebenen Kalibrierungs-Vorgang wird vorzugsweise vor einer Operation durchgeführt. Die so bestimmte Kalibrierungs-Transformation wird abgespeichert und kann dann zur Registrierung eines Instruments 500 relativ zu einem anatomischen Modell einer anatomischen Struktur eines Patienten genutzt werden.

Figur 3 zeigt einen Patienten 400 liegend im Röntgengerät 100. Von einer anatomischen Struktur 410 des Patienten 400 wird ein 3D-Röntgenscan durchgeführt. An der anatomischen Struktur 410 des Patienten ist ein Referenzstern 420 angebracht, der Marker, insbesondere passive Marker umfasst. Die passiven Marker reflektieren Licht, insbesondere Infrarotstrahlung, die von einem Infrarotsender (nicht gezeigt) emittiert werden. Die Marker können auch aktiv ausgebildet sein, so dass sie Licht ausstrahlen. Die Marker werden von der Kamera 200 des Navigationssystems detektiert. Die Kamera 200 detektiert auch Marker, die am Kit 50 angebracht sind, um so die Lage des Kit 50 zu erkennen.

Durch Detektion des Kits 50 bei einer bekannten Position der Röntgeneinheit 110, 120, 130 (z.B. Startposition) oder eines am Teil 150 angebrachten Referenzsterns 22 lässt sich bestimmen, ob sich die Lage des Röntgengeräts 100 im Vergleich zum vorher in Verbindung mit Figur 1 und 2 beschriebenen Kalibriervorgang relativ zur Kamera 200 geändert hat. Insbesondere lässt sich bestimmen, ob sich die Lage des Röntgengerät-Bezugssystems 70, also z.B. die Lage des Kits 50 in der Scanstartposition oder die Lage des Bezugssystems 20, geändert hat. Da das Bezugssystem 20 im Bezugssystem 70 ruht, kann auch das Bezugssystem 20 als ein Beispiel für ein Röntgengerät-Bezugssystem angesehen werden. Auch lässt sich bestimmen, ob sich die Lage des Scanpfades, entlang dem die Röntgenquelle 120 und der Röntgenstrahldetektor 130 während des 3D-Scans bewegt werden, relativ zur Kamera 200 geändert hat. Auf diese Art und Weise lässt sich eine Transformation bestimmen, die die Lageänderung des Röntgengerät-Bezugssystems 70 oder auch des Scanpfades beschreibt.

Geht man davon aus, dass ein dreidimensionaler Röntgenscan (Mess-3D-Röntgenscan) der anatomischen Struktur 410 durchgeführt wurde, so kann man mathematisch, insbesondere numerisch ein dreidimensionales anatomisches Modell der anatomischen Struktur 410 erzeugen, wie dies in analoger Weise bei der Bestimmung des Kalibrier-Modells aus dem Kalibrier-3D-Röntgenscan erfolgt ist. Dieses anatomische Modell liegt im 3D-Scan-Bezugssystem 60 des Röntgengeräts vor.

## Patentansprüche

1. Verfahren zum Bestimmen einer Kalibrier-Information, die Information über eine räumliche Beziehung zwischen einem Röntgengerät-Bezugssystem (20, 70) und einem 3D-Scan-Bezugssystem (60) umfasst, wobei die bestimmte Kalibrier-Information insbesondere zur bildgestützten Navigation verwendet werden kann,

mit folgenden Schritten:

- ein 3D-Kalibrierdatensatz wird bereitgestellt, der ein dreidimensionales Kalibrier-Modell eines Kalibrier-Objekts (10) in dem 3D-Scan-Bezugssystem (60) darstellt, wobei das Kalibrier-Modell aus durch einen Kalibrier-3D-Röntgenscan erzeugten Scandaten bestimmt wurde, wobei bei dem Kalibrier-3D-Röntgenscan eine Röntgeneinheit (110, 120, 130) entlang eines 3D-Scanpfades relativ zum Kalibrier-Objekt (10) bewegt wird, wobei zumindest ein Abschnitt des 3D-Scanpfades im Röntgengerät-Bezugssystem (20, 70) ruht;
- Röntgenquellen-Relativlagedaten werden bereitgestellt, die Informationen über die relative Lage einer Röntgenquelle (122) relativ zu einer an der Röntgeneinheit (110, 120, 130) angebrachten Röntgeneinheit-Markereinrichtung (50) bei Erstellung von realen zweidimensionalen Röntgenbildern umfassen;
- ein 2D-Kalibrierdatensatz wird bereitgestellt, der ein erstes der realen zweidimensionalen Röntgenbilder beschreibt, das mittels Durchstrahlung zumindest des Kalibrier-Objekts (10) bei einer ersten Lage (Fig. 1) der Röntgenquelle (122) im Röntgengerät-Bezugssystem (20, 70) erzeugt wurde und der weiterhin

  a) ein zweites der realen zweidimensionalen Röntgenbilder beschreibt, das mittels Durchstrahlung zumindest des im Vergleich zum ersten zweidimensionalen Röntgenbild ortsfesten Kalibrier-Objekts (10) bei einer zweiten Lage (Fig. 2) der Röntgenquelle (122) im Röntgengerät-Bezugssystem erzeugt wurde und/oder
  b) die Abmessungen des Kalibrier-Objekts (10) beschreibt;

- ein Röntgeneinheit-Datensatz wird bereitgestellt, der die Lage der Röntgeneinheit-Markereinrichtung (50) im Röntgengerät-Bezugssystem (20, 70) bei der Erzeugung des mindestens einen realen zweidimensionalen Röntgenbildes beschreibt;

**dadurch gekennzeichnet, dass**
basierend auf den Röntgenquellen-Relativlagedaten, dem Röntgeneinheit-Datensatz und dem 2D-Kalibrierdatensatz mittels eines Anpassungsverfahrens die Kalibrier-Information bestimmt wird, wobei das Anpassungsverfahren mindestens ein virtuelles zweidimensionales Röntgenbild des Kalibrier-Modells an mindestens eines der realen zweidimensionalen Röntgenbilder anpasst, wobei ermittelt wird, wie das Kalibrier-Objekt (10) im Röntgengerät-Bezugssystem (20, 70) liegen müsste, um das mindestens eine reale zweidimensionale Röntgenbild zu erzeugen.

2. Verfahren nach Anspruch 1, bei welchem das virtuelle zweidimensionale Röntgenbild bestimmt wird, indem das Kalibrier-Modell virtuell mit Röntgenstrahlen durchstrahlt wird, die von einer virtuellen Röntgenquelle ausgehen, die sich in der ersten Lage befindet, wobei die Kalibrier-Information eine Kalibrier-Transformation umfasst, die so bestimmt wird, dass sich mittels der Kalibrier-Transformation die Lage des Kalibrier-Modells so transformieren lässt, dass sich für die transformierte Lage die Anpassung ergibt und/oder bei welchem zum Ermitteln der virtuellen zweidimensionalen Röntgenbilder das Kalibrier-Modell virtuell von Röntgenstrahlen einer virtuellen Röntgenquelle durchstrahlt wird, die dieselbe Lagen einnimmt, wie die Lagen der realen Röntgenquelle bei Erzeugung der realen zweidimensionalen Röntgenbilder.

3. Verfahren nach Anspruch 2, bei welchem die Kalibrier-Transformation basierend auf einer als gültig befundenen Test-Transformation, die die Lage des Kalibrier-Modells im 3D-Scan-Bezugssystem (60) in die Lage des Kalibrier-Modells im Röntgengerät-Bezugssystem (20, 70) transformiert, bestimmt wird, wobei hierzu die Test-Transformation geändert wird, bis mittels der Test-Transformation eine Übereinstimmung zwischen den virtuellen und realen Röntgenbildern in einem vorbestimmen Umfang erzielt wird, wobei die so bestimmte Test-Transformation die als gültig befundene Test-Transformation ist.

4. Verfahren nach Anspruch 3, bei welchem die Test-Transformation einen rotatorischen und einen translatorischen Anteil umfasst, wobei die Anteile zum Bestimmen der Kalibrier-Transformation geändert werden.

5. Verfahren nach einem der vorhergehenden Ansprüche, bei welchem die Röntgenquellen-Relativlagedaten aus mindestens einem realen zweidimensionalen Röntgenbild gewonnen werden, das mittels Durchstrahlung einer Röntgenquellen-Bestimmungshilfe (140) mit Röntgenstrahlen von der Röntgenquelle erzeugt wurde, als auch aus der bekannten relativen Lage der Röntgeneinheit-Markereinrichtung (50) und der Röntgenquellen-Bestimmungshilfe (140), die bei der Erzeugung des realen zweidimensionalen Röntgenbildes gegeben war.

6. Verfahren nach einem der vorhergehenden Ansprüche, bei welchem ein 3D-Messdatensatz bereitgestellt wird, der ein dreidimensionales anatomisches Modell einer durch einen Mess-3D-Röntgenscan erfassten anatomischen Struktur (410) im 3D-Scan-Bezugssystem beschreibt.

7. Verfahren nach Anspruch 6, bei welchem Gegenstands-Röntgeneinheit-Daten bereitgestellt werden, die die Lage eines Gegenstands (500) im Röntgengerät-Bezugssystem (20, 70) beschreiben, wobei basierend auf den Gegenstands-Röntgeneinheit-Daten und der Kalibrier-Transformation die Lage des Gegenstands (500) relativ zu dem dreidimensionalen anatomischen Modell bestimmt wird.

8. Verfahren nach Anspruch 6 oder 7, bei welchem Gegenstands-Patienten-Daten bereitgestellt werden, die die Lage ($V_{80}$) eines Gegenstands (500) in einem Patienten-Bezugssystem (80) beschreiben, in dem eine ortsfest mit der anatomischen Struktur (410) verbundene Patienten-Markereinrichtung (420) ruht, bei welchem Markerrelativlagedaten bereitgestellt werden, die die Lage der Patienten-Markereinrichtung (420) im Röntgengerät-Bezugssystem (20, 70) beschreiben, wobei basierend auf den Markerrelativlagedaten eine Mess-Transformation ($M_{80\text{-}70}$) bestimmt wird, wobei basierend auf den Gegenstands-Patienten-Daten mittels der Mess-Transformation ($M_{80\text{-}70}$) und der Kalibrier-Transformation ($M_{70\text{-}60}$) die Lage ($V_{60}$) des Gegenstands (500) relativ zu dem dreidimensionalen anatomischen Modell bestimmt wird.

9. Verfahren nach einem der vorhergehenden Ansprüche, bei welchem der 3D-Kalibrierdatensatz ein unregelmäßig geformtes Kalibrier-Objekt beschreibt, das in zweidimensionalen Röntgenbildern Konturen zeigt.

10. Programm, das, wenn es in einen Datenverarbeitungsvorrichtung geladen wird oder auf einer Datenverarbeitungsvorrichtung läuft, die Datenverarbeitungsvorrichtung veranlasst, das Verfahren nach einem der Ansprüche 1 bis 9 auszuführen.

11. Speichermedium, auf dem das Programm nach Anspruch 10 gespeichert ist, oder Signalwelle, die Information trägt, die das Programm nach Anspruch 10 darstellt.

12. Datenverarbeitungsvorrichtung (300) zum Bestimmen der Lage eines einem 3D-Röntgenscan unterzogenen Kalibrier-Objekts (10) in einem Navigations-Bezugssystem (30), wobei in die Datenverarbeitungsvorrichtung (300) das Programm nach Anspruch 10 geladen ist oder auf der Datenverarbeitungsvorrichtung (300) das Programm nach Anspruch 10 läuft.

13. Navigationssystem
mit der Datenverarbeitungsvorrichtung (300) nach Anspruch 12 und
mit einer Detektionseinrichtung zum Detektieren der Röntgeneinheit-Markereinrichtung (50) und zum Zuführen von Marker-Detektionssignalen zu der Datenverarbeitungsvorrichtung (300).

14. Navigationssystem nach Anspruch 13 mit einem Röntgengerät (100) zum Zuführen von Röntgenstrahl-Detektionssignalen zu der Datenverarbeitungsvorrichtung (300), wobei das Röntgengerät eine Röntgenstrahldetektionseinrichtung (130) umfasst.

**Claims**

1. A method for determining calibration information which includes information on a spatial relationship between an x-ray apparatus reference frame (20, 70) and a three-dimensional scan reference frame (60), wherein the calibration information determined can in particular be used for image-guided navigation, comprising the steps of:

 • providing a three-dimensional calibration data set which represents a three-dimensional calibration model of a calibration object (10) in the three-dimensional scan reference frame (60), wherein the calibration model has been determined from scan data generated by a three-dimensional calibration x-ray scan, wherein in the three-dimensional calibration x-ray scan, an x-ray unit (110, 120, 130) is moved along a three-dimensional scanning path relative to the calibration object (10), wherein at least a portion of the three-dimensional scanning path lies in the x-ray apparatus reference frame (20, 70);
 • providing x-ray source relative position data which include information on the relative position of an x-ray source (122) relative to an x-ray unit marker device (50) attached to the x-ray unit (110, 120, 130) when actual two-dimensional x-ray images are produced;
 • providing a two-dimensional calibration data set which describes a first of the actual two-dimensional x-ray images which has been produced by means of irradiating at least the calibration object (10) in a first position (Figure 1) of the x-ray source (122) in the x-ray apparatus reference frame (20, 70), and which also describes:

 a) a second of the actual two-dimensional x-ray images which has been produced by means of irradiating at least the calibration object (10), which is spatially fixed as compared to the first two-dimensional x-ray image, in a second position (Figure 2) of the x-ray source (122) in the x-ray apparatus reference frame; and/or

 b) the dimensions of the calibration object (10);

 • providing an x-ray unit data set which describes the position of the x-ray unit marker device (50) in the x-ray apparatus reference frame (20, 70) when the at least one actual two-dimensional x-ray image is generated;
 **characterised in that**
 the calibration information is determined by means of an adapting method on the basis of the x-ray source relative position data, the x-ray unit data set and the two-dimensional calibration data set,
 wherein the adapting method adapts at least one virtual two-dimensional x-ray image of the calibration model to at least one of the actual two-dimensional x-ray images, wherein it is ascertained how the calibration object (10) would have to lie in the x-ray apparatus reference frame (20, 70) in order to generate the at least one actual two-dimensional x-ray image.

2. The method according to claim 1, wherein the virtual two-dimensional x-ray image is determined by virtually irradiating the calibration model with x-ray beams emitted from a virtual x-ray source which is situated in the first position, wherein the calibration information includes a calibration transformation which is determined in such a way that the position of the calibration model can be transformed by means of the calibration transformation, such that the adaptation results for the transformed position, and/or wherein in order to ascertain the virtual two-dimensional x-ray images, the calibration model is virtually irradiated by x-ray beams of a virtual x-ray source which assumes the same positions as the positions of the actual x-ray source when the actual two-dimensional x-ray images are generated.

3. The method according to claim 2, wherein the calibration transformation is determined on the basis of a test transformation which is found to be valid and which transforms the position of the calibration model in the three-dimensional scan reference frame (60) into the position of the calibration model in the x-ray apparatus reference frame (20, 70), wherein in this respect, the test transformation is changed until a match between the virtual and actual x-ray images is achieved to a predetermined extent by means of the test transformation, wherein the test transformation thus determined is the test transformation which is found to be valid.

4. The method according to claim 3, wherein the test transformation includes a rotational and a translational component, wherein the components are changed in order to determine the calibration transformation.

5. The method according to any one of the preceding claims, wherein the x-ray source relative position data are obtained from at least one actual two-dimensional x-ray image which has been generated by means of irradiating an x-ray source determining aid (140) with x-ray beams from the x-ray source, and from the known relative position of the x-ray unit marker device (50) and the x-ray source determining aid (140) which was given when the actual two-dimensional x-ray image was generated.

6. The method according to any one of the preceding claims, wherein a three-dimensional measurement data set is provided which describes a three-dimensional anatomical model of an anatomical structure (410), captured by a three-dimensional measurement x-ray scan, in the three-dimensional scan reference frame.

7. The method according to claim 6, wherein:

   object/x-ray unit data are provided which describe the position of an object (500) in the x-ray apparatus reference frame (20, 70); the position of the object (500) relative to the three-dimensional anatomical model is determined on the basis of the object/x-ray unit data and the calibration transformation.

8. The method according to claim 6 or 7, wherein:

   object/patient data are provided which describe the position ($V_{80}$) of an object (500) in a patient reference frame (80), in which a patient marker device (420) lies which is connected, spatially fixed, to the anatomical structure (410); marker relative position data are provided which describe the position of the patient marker device (420) in the x-ray apparatus reference frame (20, 70); a measurement transformation ($M_{80-70}$) is determined on the basis of the marker relative position data; the position ($V_{60}$) of the object (500) relative to the three-dimensional anatomical model is determined on the basis of the object/patient data by means of the measurement transformation ($M_{80-70}$) and the calibration transformation ($M_{70-60}$).

9. The method according to any one of the preceding claims, wherein the three-dimensional calibration data set describes an irregularly shaped calibration object which shows contours in two-dimensional x-ray images.

10. A program which, when it is loaded onto a data processing device or is running on a data processing device, causes the data processing device to perform the method according to any one of claims 1 to 9.

11. A storage medium on which the program according to claim 10 is stored, or a signal wave which carries information representing the program according to claim 10.

12. A data processing device (300) for determining the position of a calibration object (10), which is subject to a three-dimensional x-ray scan, in a navigation reference frame (30), wherein the program according to claim 10 is loaded onto the data processing device (300) or the program according to claim 10 is running on the data processing device (300).

13. A navigation system, comprising:

   the data processing device (300) according to claim 12; and a detection device for detecting the x-ray unit marker device (50) and for feeding marker detection signals to the data processing device (300).

14. The navigation system according to claim 13, comprising an x-ray apparatus (100) for feeding x-ray beam detection signals to the data processing device (300), wherein the x-ray apparatus includes an x-ray beam detection device (130).

**Revendications**

1. Procédé pour déterminer une information de calibrage incluant une information concernant une relation spatiale entre un système de référence d'appareil de radiographie (20, 70) et un système de référence de balayage en 3D (60), dans lequel l'information de calibrage déterminée peut être notamment utilisée pour la navigation assistée par imagerie, comportant les étapes suivantes consistant à :

   • fournir un ensemble de données de calibrage en 3D qui représente un modèle de calibrage tridimensionnel d'un objet de calibrage (10) dans le système de référence de balayage en 3D (60), le modèle de calibrage ayant été déterminé à partir de données de balayage générées par un balayage de rayons X de calibrage en 3D, une unité de radiographie (110, 120, 130) étant déplacée le long d'un trajet de balayage en 3D par rapport à l'objet de calibrage (10) pendant le balayage de rayons X de calibrage en 3D, au moins une portion du trajet de balayage en 3D se situant dans le système de référence de l'appareil de radiographie (20, 70),

• fournir des données de position relative de source de rayons X qui incluent des informations concernant la position relative d'une source de rayons X (122) par rapport à un marqueur d'unité de radiographie (50) fixé sur l'unité de radiographie (110, 120, 130) lors de la production d'images radiographiques bidimensionnelles réelles,

• fournir un ensemble de données de calibrage en 2D qui décrit une première image parmi les images radiographiques bidimensionnelles réelles, laquelle première image a été produite en exposant au moins l'objet de calibrage (10) à un rayonnement dans une première position (figure 1) de la source de rayons X (122) dans le système de référence d'appareil de radiographie (20, 70) et, qui décrit en outre

a) une seconde image parmi les images radiographiques bidimensionnelles réelles, laquelle seconde image a été produite en exposant à un rayonnement au moins l'objet de calibrage (10), spatialement fixe par rapport à la première image radiographique bidimensionnelle, dans une seconde position (figure 2) de la source de rayons X (122) dans le système de référence d'appareil de radiographie et/ou

b) les dimensions de l'objet de calibrage (10),

• fournir un ensemble de données d'unité de radiographie qui décrit la position du marqueur d'unité de radiographie (50) dans le système de référence d'appareil de radiographie (20, 70) lors de la production de la au moins une image radiographique bidimensionnelle réelle,

**caractérisé en ce que**

sur la base des données de position relative de source de rayons X, de l'ensemble de données de l'unité de radiographie et de l'ensemble de données de calibrage en 2D, l'information de calibrage est déterminée au moyen d'un procédé d'adaptation, le procédé d'adaptation adaptant au moins une image radiographique bidimensionnelle virtuelle du modèle de calibrage à au moins une des images radiographiques bidimensionnelles réelles, en déterminant la manière dont l'objet de calibrage (10) doit être positionné dans le système de référence d'appareil de radiographie (20, 70) afin de produire la au moins une image radiographique bidimensionnelle réelle.

2. Procédé selon la revendication 1, dans lequel l'image radiographique bidimensionnelle virtuelle est déterminée en soumettant virtuellement le modèle de calibrage à des rayons X provenant d'une source de rayons X virtuelle qui se situe dans la première position, dans lequel l'information de calibrage inclut une transformation de calibrage qui est déterminée de manière à pouvoir transformer la position du modèle de calibrage au moyen de la transformation de calibrage de telle sorte qu'il en résulte l'adaptation pour la position transformée et/ou dans lequel, pour déterminer les images radiographiques bidimensionnelles virtuelles, le modèle de calibrage est virtuellement soumis à des rayons X d'une source de rayons X virtuelle qui occupe les mêmes positions que les positions de la source de rayons X réelle lors de la production des images radiographiques bidimensionnelles réelles.

3. Procédé selon la revendication 2, dans lequel la transformation de calibrage est déterminée sur la base d'une transformation de test jugée valide qui transforme la position du modèle de calibrage dans le système de référence de balayage en 3D (60) en position du modèle de calibrage dans le système de référence d'appareil de radiographie (20, 70), dans lequel la transformation de test est changée à cet effet jusqu'à l'obtention d'une correspondance entre les images radiographiques virtuelles et réelles dans une certaine mesure prédéterminée au moyen de la transformation de test, dans lequel la transformation de test ainsi déterminée est la transformation de test jugée valide.

4. Procédé selon la revendication 3, dans lequel la transformation de test inclut une composante de rotation et une composante de translation, dans lequel les composantes sont changées afin de déterminer la transformation de calibrage.

5. Procédé selon l'une des revendications précédentes, dans lequel les données de position relative de source de rayons X ont été acquises à partir d'au moins une image radiographique bidimensionnelle réelle qui a été produite en soumettant une aide à la détermination de source de rayons X (140) à des rayons X provenant de la source de rayons X, ainsi qu'à partir de la position relative connue du marqueur d'unité de radiographie (50) et de l'aide à la détermination de source de rayons X (140) qui a été obtenue lors de la production de l'image radiographique bidimensionnelle réelle.

6. Procédé selon l'une des revendications précédentes, dans lequel on détermine un ensemble de données de mesure en 3D qui décrit un modèle anatomique tridimensionnel d'une structure anatomique (410), détectée par un balayage de rayons X de mesure en 3D, dans le système de référence de balayage en 3D.

**7.** Procédé selon la revendication 6, dans lequel on fournit des données d'unité de radiographie/objet qui décrivent la position d'un objet (500) dans le système de référence d'appareil de radiographie (20, 70), dans lequel sur la base des données d'unité de radiographie/objet et de la transformation de calibrage, la position de l'objet (500) par rapport au modèle anatomique tridimensionnel est déterminée.

**8.** Procédé selon la revendication 6 ou 7, dans lequel on fournit des données de patient/objet qui décrivent la position ($V_{80}$) d'un objet (500) dans un système de référence de patient (80), dans lequel se situe un marqueur de patient (420) relié à une structure anatomique (410) de manière spatialement fixe, dans lequel on fournit des données de position relative de marqueur qui décrivent la position du marqueur de patient (420) dans le système de référence d'appareil de radiographie (20, 70),
dans lequel sur la base des données de position relative de marqueur, on détermine une transformation de mesure ($M_{80-70}$),
dans lequel sur la base des données de patient/objet, on détermine la position ($V_{60}$) de l'objet (500) par rapport au modèle anatomique tridimensionnel au moyen de la transformation de mesure ($M_{80-70}$) et de la transformation de calibrage ($M_{70-60}$).

**9.** Procédé selon l'une des revendications précédentes, dans lequel l'ensemble de données de calibrage en 3D décrit un objet de calibrage formé de manière irrégulière qui présente des contours dans des images radiographiques bidimensionnelles.

**10.** Programme qui, lorsqu'il est chargé dans un dispositif de traitement de données ou lorsqu'il s'exécute sur un dispositif de traitement de données, amène le dispositif de traitement de données à mettre en oeuvre le procédé selon l'une quelconque des revendications 1 à 9.

**11.** Support de mémorisation sur lequel est stocké le programme selon la revendication 10, ou onde de signal transportant des informations représentant le programme selon la revendication 10.

**12.** Dispositif de traitement de données (300) pour déterminer la position d'un objet de calibrage (10) soumis à un balayage de rayons X en 3D dans un système de référence de navigation (30), dans lequel le programme selon la revendication 10 est chargé dans le dispositif de traitement de données (300), ou dans lequel le programme selon la revendication 10 s'exécute sur le dispositif de traitement de données (300).

**13.** Système de navigation comportant le dispositif de traitement de données (300) selon la revendication 12 et un dispositif de détection pour détecter le marqueur d'unité de radiographie (50) et pour alimenter le dispositif de traitement de données (300) en signaux de détection de marqueur.

**14.** Système de navigation selon la revendication 13 comportant un appareil de radiographie (100) pour alimenter le dispositif de traitement de données (300) en signaux de détection de rayons X, dans lequel l'appareil de radiographie inclut un dispositif de détection de rayons X (130).

Fig. 1

EP 2 119 397 B1

Fig. 2

EP 2 119 397 B1

Fig. 3

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4791934 A
- DE 10215808 B4
- US 6484049 B1
- DE 102005059301 A1
- EP 1313065 A1
- DE 19936408 A1

**In der Beschreibung aufgeführte Nicht-Patentliteratur**

- **ROGER Y. TSAI.** An Efficient and Accurate Camera Calibration Technique for 3D Machine Vision. *Proceedings of IEEE Conference on Computer Vision and Pattern Recognition,* 1986, 364-374
- **ROGER Y. TSAI.** A Versatile Camera Calibration Technique for High-Accuracy 3D Machine Vision Metrology Using Off-the-Shelf TV Cameras and Lenses. *IEEE Journal of Robotics and Automation,* August 1987, vol. RA-3 (4), 323-344